# FASCICULE DE BREVET EUROPEEN

(11) **EP 1 362 021 B1**
(45) Date de publication et mention de la délivrance du brevet: **05.08.2009**
(21) Numéro de dépôt: 02704833.9
(22) Date de dépôt: 14.02.2002
(51) Int. Cl.: C07C 37/82, C11B 9/02

(54) **PROCEDE D'EXTRACTION, DE FRACTIONNEMENT ET DE PURIFICATION DE COMPOSES POLYPHENOLIQUES ISSUS D'ECARTS DE TRIAGE DE VEGETAUX FRAIS UTILISANT UNE RESINE A HAUT RENDEMENT D'ADSORPTION ET D'ELUTION**
VERFAHREN ZUR EXTRAHIERUNG, FRAKTIONIERUNG UND REINIGUNG MITTELS EINES HARZES MIT HOHEM ADSORPTIONS- UND ELUIERUNGSRENDEMENT, VON POLYPHENOLISCHEN VERBINDUNGEN ERHALTEN AUS AUSLESEABFÄLLEN VON FRISCHEN GEMÜSEN
METHOD FOR EXTRACTING, FRACTIONATING AND PURIFYING POLYPHENOL COMPOUNDS ORIGINATING FROM FRESH VEGETABLE CULLS USING A HIGH-ABSORPTION AND -ELUTION RESIN

(30) Priorité: 15.02.2001 FR 0102096
(43) Date de publication de la demande: 19.11.2003
(73) Titulaire: INSTITUT NATIONAL DE LA RECHERCHE AGRONOMIQUE (INRA), 75338 Paris Cédex 07 (FR); SCALIME NUTRITION, 84140 Avignon (FR)
(72) Inventeur: GOUPY, Pascale, F-84400 Gargas (FR); AMIOT-CARLIN, Marie-Josephe, F-84140 Montfavet (FR); ESCUDIER, Jean-Louis, F-11110 Armissan (FR); MIKOLAJCZAK, Michel, F-11430 Gruissan (FR); MARTIN, Michel, F-11200 Nevian (FR)
(74) Mandataire: Catherine, Alain
(86) Numéro de dépôt international: PCT/FR2002/000570
(87) Numéro de publication internationale: WO 2002/064536

(56) Documents cités:
- FR-A- 2 307 779
- "LES EXTRAITS SCANOLINE" 27 février 2007 (2007-02-27), SCALIME NUTRITION SAS (FICHE TECHNIQUE) See page 7

## Description

L'invention concerne un procédé d'extraction, de fractionnement et de purification de composés issus de plantes potagères feuillues de la famille des Composées ou à bulbe de la famille des Liliacées ou d'écarts de triage de végétaux frais, pour obtenir un extrait riche en composés polyphénoliques.

En particulier, l'invention a pour objet un procédé d'extraction, de fractionnement et de purification de composés polyphénoliques, dans lequel la purification est réalisée par adsorption des composés polyphénoliques sur une résine adsorbante, puis élution des composés retenus sur la résine.

Par extrait riche en composés polyphénoliques, on entend au sens de la demande un produit ayant une teneur minimale en composés polyphénoliques égale ou supérieure à 30% par rapport au poids sec.

Par purification, on entend au sens de la demande la récupération ou la sélection des composés phénoliques extraits.

Les procédés d'extraction et de purification de composés polyphénoliques contenus dans des matières végétales mettant en oeuvre une résine adsorbante sont connus de l'art antérieur.

Le brevet américain US-5,994,413 concerne un procédé d'extraction de composés polyphénoliques à partir de fruits de rosacées, comme les pommes, les poires, les pêches et les fruits apparentés. Ce brevet décrit plus particulièrement un procédé d'isolement et de purification de composés polyphénoliques à partir de fruits par extraction de jus à partir de fruits pelés ou non pelés, par broyage des fruits puis centrifugation et récupération, puis passage du jus ainsi obtenu sur une résine capable de retenir sélectivement les composés phénoliques, et enfin élution des polyphénols retenus sur la résine pour obtenir une poudre riche en composés polyphénoliques. Ce brevet cite divers adsorbants, tels que les résines synthétiques styrène-divinylbenzène, lés résines échangeuses d'anions ou encore des gels de silice sur lesquels sont fixés chimiquement les groupes octadécyl, l'élution des polyphénols adsorbés sur ces résines ayant lieu par addition d'une solution d'alcool, tel que l'éthanol.

Le brevet américain US-5,141,611 décrit un procédé permettant d'éliminer des substances polyphénoliques contenues dans une solution et, le cas échéant, de les récupérer en utilisant une résine polyamide sur laquelle les polyphénols sont adsorbés.

Le brevet américain US-5,856,429 concerne un procédé d'élimination des polyphénols à partir de liquides, dans lequel les polyphénols sont retenus sur une résine à base d'amide. Ce brevet divulgue également d'autres types de support adsorbant des polyphénols, tels que le Nylon ou le polyvinylpolypyrrolidone (PVPP).

Le brevet américain US-4,910,182 décrit un procédé de stabilisation de boissons contenant des polyphénols dans lequel est mis en oeuvre un support adsorbant à base de polyvinylpolypyrrolidone (PVPP). Ce procédé est particulièrement adapté à l'élimination de composés polyphénoliques à partir de la bière.

Le brevet américain US-4,008,339 concerne un procédé d'élimination des polyphénols à partir de boissons d'origine végétale telles que les bières, les vins et les jus de fruits, mettant en oeuvre une résine adsorbante à base de polyamide N-substitués.

Ces procédés présentent cependant l'inconvénient d'utiliser des résines qui possèdent soit un haut rendement d'adsorption, soit un haut rendement d'élution, mais qui ne possèdent pas à la fois un haut rendement d'adsorption et un haut rendement d'élution.

La présente invention a donc pour objet un procédé d'extraction, de fractionnement et de purification de composés polyphénoliques remédiant aux inconvénients de l'art antérieur.

Plus particulièrement, la présente invention a pour objet un procédé d'extraction, de fractionnement et de purification, comprenant une étape d'extraction des composés polyphénoliques issus de plantes potagères feuillues de la famille des Composées ou à bulbe de la famille des Liliacées ou d'écarts de triage de végétaux frais, une étape d'adsorption puis une étape d'élution de ces composés mettant en oeuvre une résine ayant des caractéristiques physiques particulières, lui conférant un haut pouvoir d'adsorption des composés polyphénoliques et une forte capacité d'élution.

L'invention a également pour objet un produit riche en composés polyphénoliques obtenu par le procédé selon l'invention.

L'invention a encore pour objet l'utilisation d'un produit tel que défini ci-dessus dans la fabrication de produits cosmétiques et/ou de composés pharmaceutiques et/ou d'ingrédients alimentaires.

Les buts de la présente invention sont donc atteints par un procédé d'extraction, de fractionnement et de purification de composés polyphénoliques issus de plantes potagères feuillues de la famille des Composées ou à bulbe de la famille des Liliacées ou d'écarts de triage de végétaux frais, comprenant les étapes suivantes :
- a) extraction des composés polyphénoliques pour obtenir un extrait brut de végétaux,
- b) adsorption sur une résine adsorbante des composés polyphénoliques contenus dans l'extrait brut,
- c) élution des composés polyphénoliques retenus sur la résine pour obtenir un extrait purifié,
- d) concentration puis séchage éventuel de l'extrait purifié pour obtenir un produit riche en composés polyphénoliques,

Ce procédé est **caractérisé en ce que** la résine est une résine styrène-divinylbenzène présentant les caractéristiques physiques suivantes :
- 1) des pores ayant une taille moyenne allant de 50 à 110 Å, de préférence de 60 à 100 Å,
- 2) une surface spécifique égale ou supérieure à 800 m²/g, de préférence égale ou supérieure à 880 m²/g, conférant à la résine un pouvoir d'adsorption élevé des particules organiques,
- 3) un volume de pores supérieur à 1 ml/g, et de préférence égal ou supérieur à 1,4 ml/g.

De manière préférée, la résine selon l'invention présente les caractéristiques physiques suivantes :
- 1) des pores ayant une taille moyenne allant de 80 à 110 Å de préférence de 88 à 92 Å, et mieux d'environ 90 Å,
- 2) une surface spécifique égale ou supérieure à 1000 m²/g, de préférence égale ou supérieure à 1200 m²/g,
- 3) un volume de pores égal ou supérieur à 2 ml/g, et de préférence égal ou supérieur à 2,4 ml/g.

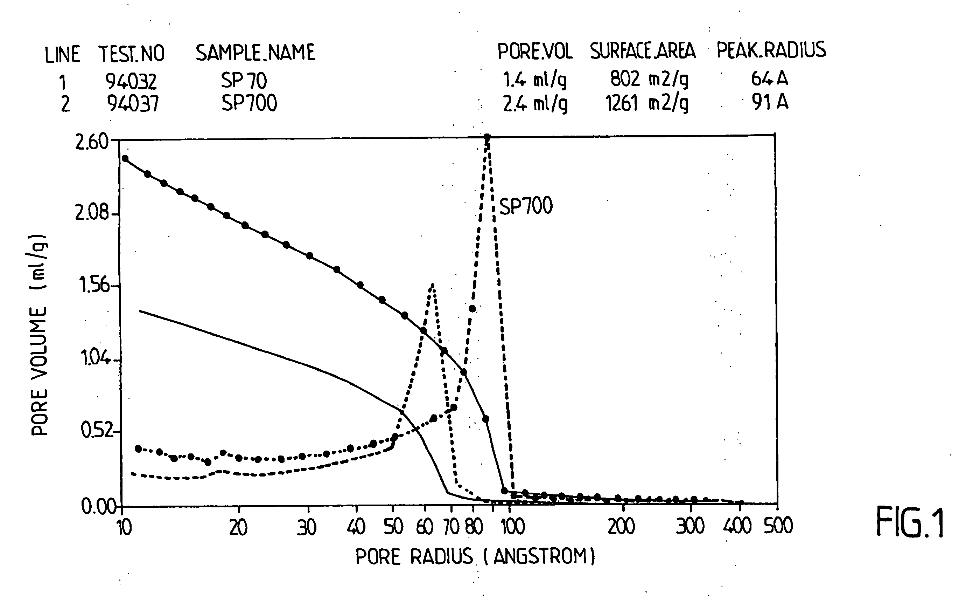

La distribution des pores en fonction de leur taille est représenté sur la figure 1, pour deux résines selon l'invention.

Les résines selon l'invention adsorbent les composés polyphénoliques contenus dans les végétaux mais n'ont pas ou peu d'affinité pour toute une gamme de molécules et solutés contenus également dans ces mêmes végétaux, qui sont en partie éliminés, tels que les sels dissous, les sucres, les polyosides, les cations, les anions, les nitrates et les nitrites.

Les végétaux sont généralement des plantes ou des fruits. En particulier, dans le cas de la présente demande, les composés polyphénoliques sont contenus dans les tissus végétaux de plantes potagères.

Par plantes potagères, on entend toute plante ou végétal dont on fait une utilisation culinaire.

Les plantes potagères particulièrement préférées sont les plantes potagères feuillues de la famille des Composées et les plantes potagères à bulbe de la famille des Liliacées.

Comme exemples de plantes de la famille des Composées, on peut citer la laitue, la chicorée et en particulier la scarole et la frisée.

Parmi les différentes variétés de laitue, on citera par exemple la romaine, la batavia, l'iceberg, etc.

Comme exemples de plantes à bulbe de la famille des Liliacées, on peut citer l'oignon, l'ail, le poireau et l'échalote.

Une fois fixées sélectivement, les molécules à valoriser, c'est-à-dire les composés polyphénoliques, sont éluées avec un solvant, de préférence alimentaire.

Par solvant alimentaire, on entend, au sens de la demande tout solvant adapté à la préparation de produits destinés à l'alimentation humaine ou animale.

De préférence, le solvant alimentaire est l'éthanol ou le méthanol.

L'élution doit être facile, rapide et aussi complète que possible pour ne pas diluer l'extrait, et donc le rendement global du procédé.

L'extrait purifié, ou éluat, est généralement concentré, et l'on obtient un produit riche en composés polyphénoliques, l'extrait pouvant être séché par atomisation, ou par lyophilisation

De préférence, la résine selon l'invention répond à la formule (I) :

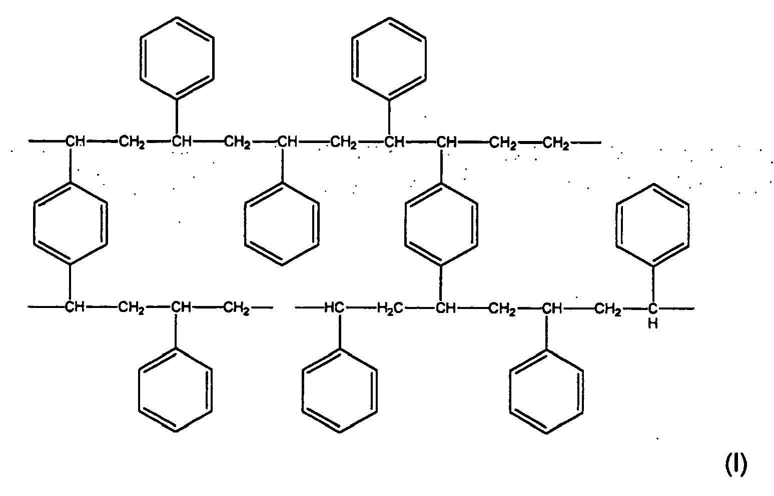

A titre d'illustration de résine selon l'invention, on citera les résines styrène divinylbenzène commercialisées par la société RESINDION sous les dénominations SP70 et SP700.

Les résines adsorbantes selon l'invention sont très sélectives vis-à-vis des composés polyphénoliques et ont une forte capacité d'élution avec des solvants alimentaires tels que le méthanol ou l'éthanol.

En particulier, le taux d'adsorption des composés polyphénoliques sur la résine est égal ou supérieur à 80%, et le taux d'élution des composés polyphénoliques est égal ou supérieur à 60%.

On entend par taux d'adsorption des composés polyphénoliques le rapport entre la quantité de composés polyphénoliques fixés sur la résine et la quantité de composés polyphénoliques contenue dans l'extrait brut.

De même, on entend par taux d'élution, le rendement global du procédé, c'est-à-dire le rapport entre la quantité de composés polyphénoliques contenus dans l'extrait purifié (ou éluat) et la quantité de composés polyphénoliques contenue dans l'extrait brut.

L'extraction des composés phénoliques comprend généralement un chauffage rapide de la plante depuis une température ambiante de 25°C jusqu'à une température pouvant atteindre 105°C, pour obtenir un jus d'exsudation chaud et un moût de plantes cuites exsudées refroidies, le jus d'exsudation constituant au moins partiellement l'extrait brut.

Le chauffage rapide est de préférence réalisé en l'absence de l'oxygène de l'air pour le blocage des activités enzymatiques et la protection des matières végétales traitées vis-à-vis d'éventuelles dégradations oxydatif ou hydrolytique.

De manière avantageuse, le chauffage est effectué en une à quinze minutes sans macération.

Pour favoriser l'extraction, c'est-à-dire pour obtenir une plus grande quantité de composés polyphénoliques dans les extraits bruts tout en respectant la qualité des composés extraits, le chauffage rapide peut être suivi par une étape de mise sous vide sensiblement immédiate de la plante, provoquant la vaporisation d'une partie de la plante, cette étape étant réalisée à une pression comprise entre 10³ et 2x10⁴ Pa.

La mise sous vide est nécessaire lorsque les composés polyphénoliques sont contenus dans des plantes potagères appartenant à la famille des Liliacées, notamment l'oignon, pour lesquelles l'extraction des composés polyphénoliques est difficile.

En revanche, lorsque les plantes sont des Composées, et notamment des salades, l'étape de mise sous vide est utile mais non nécessaire.

L'extraction des composés polyphénoliques, notamment les étapes de chauffage rapide et de mise sous vide de la matière végétale, ont été amplement décrites dans le brevet européen EP-0 728189 et dans le brevet français FR-9313286.

Différents modes de chauffage peuvent être utilisés. De préférence, le chauffage des plantes est réalisé en faisant circuler du jus provenant des plantes traitées, sur les plantes, ou de la vapeur.

D'autres procédés peuvent également être utilisés, continus ou discontinus. On citera notamment le chauffage par micro-ondes, le chauffage par induction, le chauffage ohmique, le chauffage discontinu en cuve ou le chauffage à partir d'un échangeur thermique.

Comme exemples d'échangeurs thermiques, on peut citer par exemple les échangeurs tubulaires ou à plaques, les échangeurs à surface raclée qui permettent un apport direct de calories aux plantes, les échangeurs corugais, les échangeurs coaxiaux.

L'extrait purifié peut être utilisé sous forme sèche ou sous forme liquide, mais il est préférentiellement utilisé sous forme sèche.

Selon l'invention, l'extrait purifié peut être séché par atomisation ou par lyophilisation.

Selon l'invention, l'extrait purifié a une teneur en composés polyphénoliques égale ou supérieure à 30%.

Si les plantes potagères dont sont extraits les composés polyphénoliques sont des salades, les flavonols représentent au plus 25% des composés polyphénoliques et les esters d'acides hydroxycinnamiques représentent au moins 50% des composés polyphénoliques, par rapport au poids total de l'extrait purifié sec.

Les esters d'acides hydroxycinnamiques sont notamment des dérivés de l'acide caféique comprenant les esters de l'acide tartrique avec l'acide monocaféoyltartrique, les isomères de l'acide dicaféoyltartrique, l'acide 5'-caféoylquinique (ou acide chlorogénique), l'acide 3'-5'dicaféoylquinique (ou acide isochlorogénique), l'acide caféoylmalique et l'acide féruloylquinique.

Les flavonols comprennent le kaempférol-3-glucoside (kaempférol ou lutéoline), le kaempférol-3-glucuronide, le kaempférol-3-O-malonylglucoside, la quercétine-3-glucuronide, la quercétine-3-O-malonylglucoside et les dérivés de kaempférol-malonylglucoside.

En revanche, si les plantes potagères sont des oignons, les flavonols représentent au moins 90% des composés polyphénoliques par rapport au poids total de l'extrait purifié sec.

Ils comprennent notamment la quercétine-3,4'-diglucoside, la quercétine-4'-monoglucoside, la quercétine, l'isorhamnétine-3,4'-diglucoside et l'isorhamnétine-4'-monoglucoside.

Les propriétés antiradicalaires des extraits purifiés de salade et d'oignon sont évalués par leur capacité à neutraliser le radical DDPH^{*}, selon la méthode décrite dans l'article de P. Goupy, M. Hugues, P. Boivin et M. J. Amiot, Journal of the Science of Food and Agriculture, 1999, 79, pages 1625-1634,

L'activité antiradicalaire est définie par le rapport 1/CI₅₀, où CI₅₀ est défini comme étant la quantité de produit purifié nécessaire à la réduction de 50% de la quantité de DDPH^{*} présent à une concentration initiale de 6x10⁻⁵ mole.I⁻¹.

Plus la valeur du rapport 1/CI₅₀ est élevée, plus l'activité antiradicalaire de l'extrait est importante.

Selon l'invention, l'extrait de salades a une activité antiradicalaire définie par un rapport 1/CI₅₀ égal ou supérieur à 0,2 et l'extrait d'oignons a une activité antiradicalaire définie par un rapport 1/CI₅₀ égal ou supérieur à 0,12, pour une teneur en composés polyphénoliques égale ou supérieure à 30%.

Les produits obtenus selon l'invention peuvent être utilisés pour la fabrication de produits cosmétiques, et/ou de composés pharmaceutiques, et/ou d'ingrédients alimentaires.

Les exemples suivants illustrent l'invention sans toutefois en limiter la portée.

Dans les exemples, sauf indication contraire, toutes les quantités sont exprimées en fraction massique par rapport à la quantité de composés polyphénoliques contenus dans un extrait brut.

### 1. MATIERES PREMIERES

### Extraction de composés polyphénoliques contenus dans les salades et les oignons

Des extraits de salades et d'oignons ont été préparés lors de l'étape d'extraction du procédé selon l'invention, qui est dans le brevet européen EP-0728189 et dans le brevet français FR-9313286.

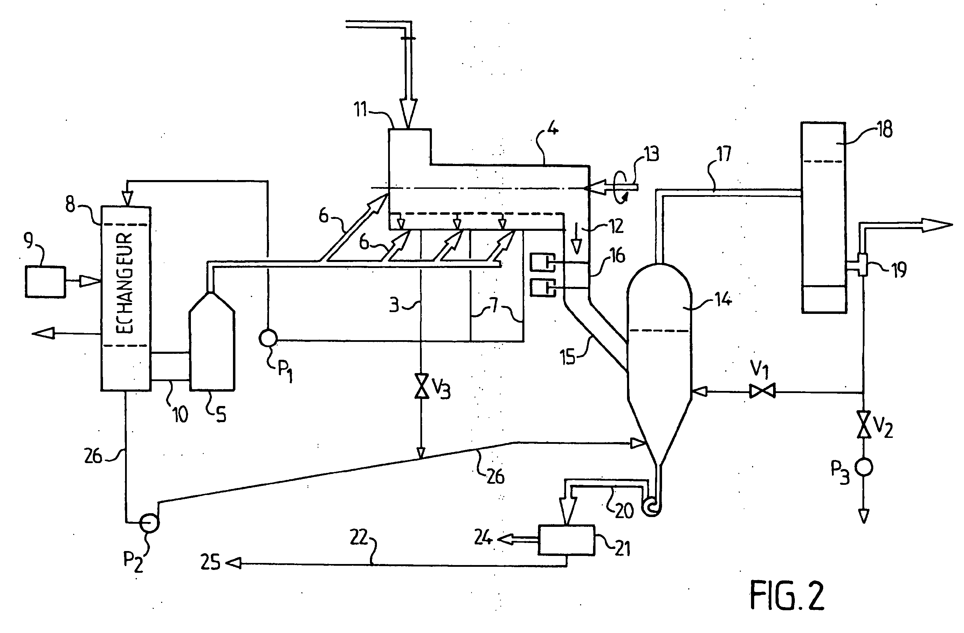

La Figure 2 représente un dispositif pour la mise en oeuvre de ce procédé.

La matière traitée par ce procédé d'extraction est constituée par des écarts de triage de salades ou d'oignons.

Le procédé peut être appliqué à des écarts de triage de végétaux frais de toute nature.

Dans le procédé illustré sur la figure 2, les écarts de triage de végétaux frais (ou matière végétale) sont amenés dans une chambre de chauffage 4.

Le chauffage de la matière végétale s'effectue à une température contrôlée précisément à l'intérieur de la gamme de température 25°C-105°C, en évitant la présence d'air qui pourrait provoquer des dégradations oxydatives.

Le chauffage de la matière végétale dans la chambre de chauffage 4 peut être effectué par tout moyen approprié. On peut par exemple, introduire dans la chambre 4 de la vapeur condensante biologique émise à partir de jus ou de condensats provenant du procédé. Cette vapeur est émise à partir du dispositif 5, par des conduits 6.

Les condensats et les jus d'égouttage ou d'exsudation sont récupérés de la chambre de chauffage 4 et transmis, par des conduits 3 et 7 et une pompe P1, dans un échangeur 8. On peut également prévoir de transmettre du jus issu de la presse (référence 22) dans l'échangeur 8. Cette variante n'est pas illustrée sur la Figure 2.

De préférence, le conduit 3 est relié au conduit 26 par une vanne V3. Ainsi, lorsque les conduits 7 véhiculent des condensats et des jus en quantité suffisante, la vanne V3 est ouverte et les condensats et les jus du conduit 3 sont directement transmis dans la chambre de détente 14. Ceci permet d'obtenir un meilleur rendement énergétique dans la mesure où les condensats et les jus véhiculés par le conduit 8 sont plus froids que ceux des conduits 7. Dans le cas contraire, la vanne V3 est fermée et l'ensemble des condensats et des jus récupérés dans les conduits 3 et 7 est transmis à l'échangeur 8.

Un générateur de vapeur 9 est relié à l'échangeur 8 qui est, de préférence, un échangeur à surface raclée transmettant de la vapeur au dispositif 5 par des moyens appropriés 10.

Ce dispositif permet d'éviter le chauffage par une vapeur d'eau exogène provenant directement d'une chaufferie.

La durée du chauffage est réglable de telle sorte qu'il est possible de chauffer soit la totalité des végétaux, soit la pellicule de ces végétaux, notamment dans le cas des oignons, soit encore une partie périphérique de ces mêmes végétaux. Ceci permet de contrôler le niveau d'extraction des différents composés polyphénoliques présents dans ces matières végétales.

La durée du chauffage est relativement courte. Elle est inférieure à 5 minutes, et de préférence égale à 3 minutes environ dans le cas de l'extraction de composés polyphénoliques contenus dans des salades. Dans le cas de l'extraction de composés polyphénoliques de l'oignon, la durée du chauffage est inférieure à 15 minutes, et de préférence égale à 10 minutes environ.

La matière végétale est chauffée depuis une température ambiante de 25°C jusqu'à une température pouvant atteindre 105°C selon la nature des végétaux : pour les salades et les oignons, la température de chauffage est de préférence supérieure ou égale à 90°C.

D'après la Figure 2, la matière végétale est transférée depuis l'entrée de la chambre de chauffage 4 jusqu'à la sortie 12, par l'intermédiaire de vis, portant des auges perforées qui sont destinées à recevoir une quantité déterminée de matière. Les vis tournent autour d'un axe 13. La vitesse de rotation, la hauteur de la couche réglable, la longueur des vis ainsi que la largeur de bande de chauffage, définissent le débit et le temps de séjour des produits chauffés.

La matière végétale chauffée est transférée depuis la sortie 12 de la chambre de chauffage 4 jusqu'à une chambre de détente 14 par l'intermédiaire d'un conduit 15. Celui-ci est équipé d'un sas ou d'une pompe 16, de préférence sensiblement étanche, de façon à éviter des fuites de vapeur.

La chambre de détente 14 est reliée par des moyens appropriés 17, à un condenseur 18 qui est lui-même relié à un dispositif de vide 19.

La matière préchauffée est mise sous vide sensiblement immédiatement. La pression à l'intérieur de la chambre de détente 14 est comprise entre 10³ et 2x10⁴ Pa absolus. La valeur de la pression est choisie en fonction de la nature des végétaux. Ainsi dans le cas des composés polyphénoliques extraits d'oignons, la pression absolue dans la chambre de détente est de 12x10³ Pa absolus, alors qu'il est avantageux d'utiliser une pression relativement plus faible, d'environ 6x10³ Pa absolus dans le cas de composés polyphénoliques extraits de salades.

Le vide dans la chambre 14 se fait par condensation des vapeurs émises à travers le condensateur 18.

Cette mise sous vide très rapide provoque une auto-évaporation des fractions liquides de la matière végétale qui déstructure la matière végétale préalablement chauffée. Ceci permet d'augmenter la mise en solution de différents composés et notamment les composés polyphénoliques.

Cet effet est d'autant plus important que la différence entre la température de chauffage de la matière végétale et celle provoquée par la mise sous vide est également importante.

La matière végétale traitée est ensuite évacuée de la chambre de détente 14 par des moyens appropriés 20. Le produit qui sort de la chambre de détente 14 est une purée de matière végétale.

Il faut noter que cette purée sort de la chambre de détente 14 à une température relativement basse, de l'ordre de 20 à 25°C, lorsque le vide présent dans cette chambre est très poussé (de l'ordre de 2x10³ Pa absolus) et que la matière végétale a été chauffée à une température appropriée.

On peut laisser la purée diffuser.

La purée est généralement pressée dans le dispositif 21, de façon à obtenir un jus, également appelé jus brut de pressurage qui s'écoule par le conduit 22.

On récupère la purée pressée en 24, qui constitue alors un gâteau de presse de matière végétale.

Si l'on souhaite uniquement obtenir un jus à partir de la purée, on récupère l'ensemble du jus obtenu par le procédé. Si l'on souhaite obtenir un produit fermenté (dans le cas de l'oignon par exemple), on procède à la fermentation de la purée provenant de la chambre de détente 14, après enzymage et levurage de cette purée.

On peut encore noter que le jus obtenu en sortie de l'échangeur 8 est transféré vers la chambre de détente 14, par l'intermédiaire du conduit 26 et de la pompe P2.

La vapeur émise lors de la mise sous vide est récupérée, en sortie du condenseur 18, sous la forme de condensats aromatiques. Ceux-ci peuvent être transférés du condenseur 18 vers la chambre de détente 14, la vanne V1 étant alors ouverte et la vanne V2 fermée, ce qui permet de restituer une purée d'un poids identique à celui de la matière première végétale. On peut également prévoir de transférer les condensats présents dans le condenseur 18 vers l'extérieur du dispositif, par l'intermédiaire de la pompe P3. Dans ce cas, la vanne V1 est fermée et la vanne V2 est ouverte. Cette variante du procédé permet de réaliser une préconcentration maîtrisable de la purée.

Dans cette variante, les condensats aromatiques peuvent être traités pour concentrer les arômes et séparer l'eau.

Le jus brut de pressurage et le jus d'exsudation, contenant les composés polyphénoliques à valoriser sont réunis pour constituer le jus brut ou l'extrait brut de végétaux.

Lorsque le procédé selon l'invention est mis en oeuvre avec le dispositif représenté sur la Figure 2, la matière première est traitée de façon continue.

Le procédé qui vient d'être décrit permet d'obtenir des extraits bruts de végétaux dont les composés polyphénoliques sont fractionnés et purifiés en utilisant une résine à haut rendement d'adsorption et d'élution.

### 2. METHODES DE MESURE

La résine R utilisée dans le procédé selon l'invention est une résine styrène-divinyl benzène répondant à la formule (I), et dont les caractéristiques physiques sont rassemblées dans le tableau 1.

**TABLEAU 1**

| Caractéristiques physiques | Résine R |
|---|---|
| taille moyenne des pores (A) | 90 |
| surface spécifique (m²/g) | 1200 |
| volume des pores (ml/g) | 2,4 |

### Quantification des composés polyphénoliques

Les composés polyphénoliques sont analysés par HPLC et quantifiés par étalonnage externe.

Pour les extraits de salades, les différentes teneurs sont exprimées en grammes équivalent acide chlorogénique pour les esters d'acides hydroxycinnamiques (EAC) et en grammes équivalent quercétine pour les flavonols (EQ).

Pour les extraits d'oignons, les teneurs sont exprimées en équivalent quercétine-4'-monoglucoside, ou en équivalent quercétine-3,4'-diglucoside pour les composés identifiés comme tels, ou en équivalent quercétine pour les autres flavonols.

Cette méthode présente l'avantage, outre de quantifier chaque composé présent dans l'extrait ou la solution analysée, de vérifier la présence de tous les composés polyphénoliques.

La quantification est réalisée pour :
- les extraits bruts déposés sur les résines,
- les solutions éluées lors de la charge et du rinçage à l'eau de la résine précédant l'élution proprement dite, pour vérifier la perte de composés polyphénoliques lors de cette étape.
- les composés polyphénoliques adsorbés sur la résine, et
- les extraits purifiés.

Le rapport de la quantité de composés phénoliques adsorbés sur la résine R sur la quantité de composés polyphénoliques contenus dans l'extrait brut déposé sur cette résine représente le taux T_{A} d'adsorption de composés phénoliques sur la résine.

De même, le rapport entre la quantité de composés polyphénoliques contenus dans l'extrait purifié et la quantité de composés polyphénoliques contenus dans l'extrait brut représente le taux T_{E} de composés polyphénoliques récupérés après élution au méthanol. Ce rapport représente en fait le rendement global du procédé de purification.

Enfin, le taux T_{NE} de composés polyphénoliques non éluables et fixés sur la résine est le rapport entre la quantité de composés polyphénoliques adsorbés sur la résine et non élués au cours de l'étape d'élution au méthanol ou élués lors du rinçage à l'eau ou lors de la charge, et la quantité de composés polyphénoliques contenus dans l'extrait déposé sur la résine.

Le taux T_{A} d'adsorption sur la résine de composés phénoliques est la somme des taux T_{E} et T_{NE.}

### EXEMPLE 1

### Purification d'extraits de salades

Les extraits bruts de salades ont été purifiés en utilisant respectivement le procédé selon l'invention.

Les extraits bruts de salades sont déposés sur la résine R (charge). Le volume de résine utilisé est de 25 ml, soit 1 BV (Bed Volum). Après un rinçage à l'eau avec un volume d'eau de 125 ml (soit 5 BV), les composés polyphénoliques sont élués par 80 ml de méthanol, soit 3,2 BV. On obtient un extrait purifié EP.

La solution éluée pendant la charge et celle éluée pendant le rinçage à l'eau de la résine sont réunies et ramenées à un volume de 350 ml, dont on analyse également la nature et la quantité de composés polyphénoliques, pour quantifier la perte de composés polyphénoliques lors de cette étape.

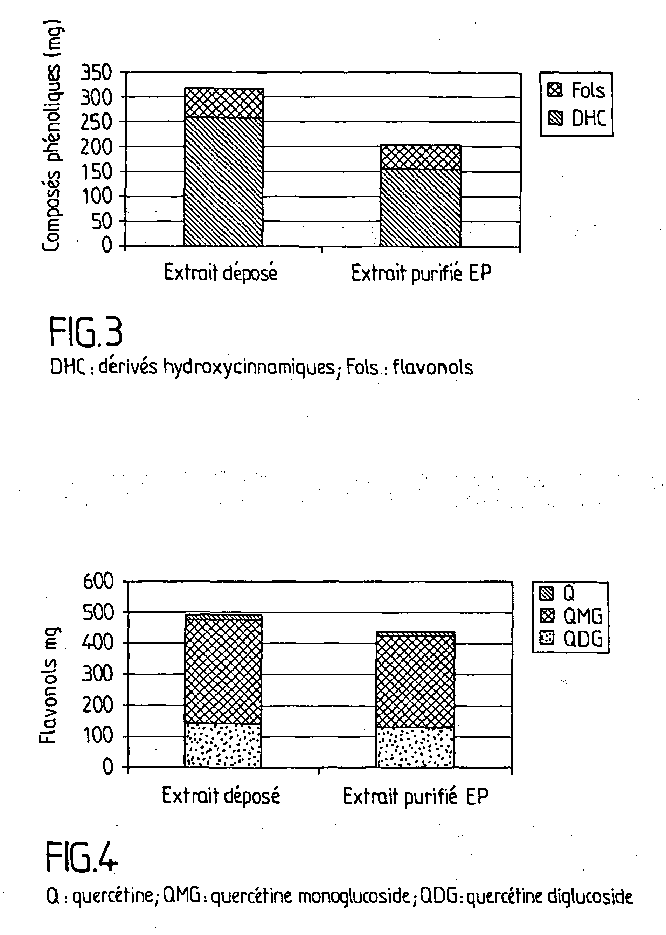

On a mesuré la quantité de composés polyphénoliques contenus dans l'extrait purifié EP. Cette mesure est représentée sur la Figure 3.

La Figure 3 montre que l'extrait brut déposé contient 317 mg de composés polyphénoliques, et l'extrait purifié EP contient 199 mg de composés phénoliques. Aucune différence qualitative n'est observée entre l'extrait déposé et l'extrait purifié : ces extraits comprennent des acides hydroxycinnamiques et des flavonols.

Les valeurs obtenues des taux T_{A}, T_{E} et T_{NE,} ainsi que la quantité de composés polyphénoliques fixés par ml de résine sont présentées dans le tableau 2.

**TABLEAU 2**

| | Résine R |
|---|---|
| T_{A} [%] | 87 |
| T_{E} [%] | 63 |
| T_{NE} [%] | 24 |
| Quantité de polyphénols fixés sur la résine [mg/ml de résine] | 11,1 |

### EXEMPLE 2

### Purification d'extraits d'oignons

Les extraits bruts d'oignons sont déposés sur la résine R Après un rinçage à l'eau (125 ml soit 5 BV), les composés sont élués par 100 ml de méthanol (soit 4 BV). On obtient un extrait purifié EP.

Les solutions éluées pendant la charge et le rinçage à l'eau des résines sont réunies et ramenées à un volume de 300 ml, pour la quantification de la perte de composés polyphénoliques lors de cette étape.

La Figure 4 montre que l'extrait déposé contient 494 mg de composés phénoliques, et l'extrait purifié EP contient 449 mg de composés phénoliques. Aucune différence qualitative n'est observée entre l'extrait déposé et l'extrait purifié EP de l'oignon qui sont à plus de 95% des flavonols dérivés de la quercétine, comprenant les composés suivants : quercétine, quercétine monoglucoside et quercétine diglucoside entre autres.

Les valeurs obtenues des taux T_{A}, T_{E} et T_{NE}, ainsi que la quantité de flavonols fixés par ml de résine sont présentées dans le tableau 3.

**TABLEAU 3**

| | EP |
|---|---|
| T_{A} [%] | 99 |
| T_{E} [%] | 91 |
| T_{NE} [%] | 9 |
| Quantité de flavonols fixés sur la résine [mg/ml de résine] | 19,6 |

Le tableau 3 montre que le taux de flavonols adsorbés sur la résine selon l'invention R et récupérés après élution au méthanol est élevé : 91 % des composés sont retenus sur l'adsorbant et élués par le méthanol.

## Revendications

1. Procédé d'extraction, de fractionnement et de purification de composés polyphénoliques issus de plantes potagères feuillues de la famille des Composées ou à bulbe de la famille des Liliacées ou d'écarts de triage de végétaux frais, comprenant les étapes suivantes :
• a) extraction des composés polyphénoliques pour obtenir un extrait brut de végétaux,
• b) adsorption sur une résine adsorbante des composés polyphénoliques contenus dans l'extrait brut,
• c) élution des composés polyphénoliques retenus sur la résine pour obtenir un extrait purifié,
• d) concentration puis séchage éventuel de l'extrait purifié pour obtenir un produit riche en composés polyphénoliques,
**caractérisé en ce que** la résine est une résine styrène-divinylbenzène présentant les caractéristiques physiques suivantes :
• 1) des pores ayant une taille moyenne allant de 50 à 110 Å, de préférence de 60 à 100 Å,
• 2) une surface spécifique égale ou supérieure à 800 m²/g, de préférence égale ou supérieure à 880 m²/g,
• 3) un volume de pores supérieur à 1 ml/g, et de préférence égal ou supérieur à 1,4 ml/g.

2. Procédé selon la revendication 1, **caractérisé en ce que** la résine présente les caractéristiques physiques suivantes:
• 1) des pores ayant une taille moyenne allant de 80 à 110 Å, de préférence de 88 à 92 Å, et mieux d'environ 90 Å,
• 2) une surface spécifique égale ou supérieure à 1000 m²/g, de préférence égale ou supérieure à 1200 m²/g,
• 3) un volume de pores égal ou supérieur à 2 ml/g, et de préférence égal ou supérieur à 2,4 ml/g.

3. Procédé selon la revendication 1 ou 2, **caractérisé en ce que** le taux d'adsorption des composés polyphénoliques sur la résine est égal ou supérieur à 80 %, et le taux d'élution est égal ou supérieur à 60%.

4. Procédé selon l'une quelconque des revendications précédentes, **caractérisé en ce que** la résine répondant à la formule (I) :

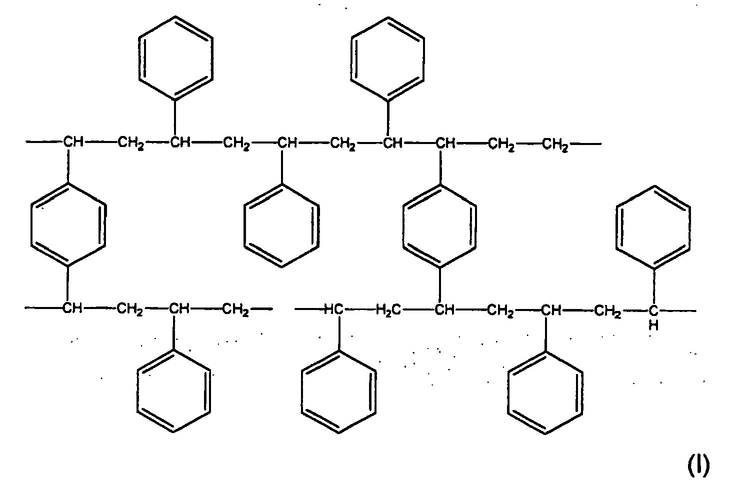

5. Procédé selon l'une quelconque des revendications précédentes, **caractérisé en ce que** l'étape (a) d'extraction comprend une étape de chauffage rapide de la plante depuis une température ambiante de 25°C jusqu'à une température de 105°C pour obtenir un jus d'exsudation chaud et un moût de plantes cuites exsudées refroidies, le jus d'exsudation constituant au moins partiellement l'extrait brut.

6. Procédé selon la revendication 5, **caractérisé en ce que** le chauffage est effectué en moins de quinze minutes, sans macération.

7. Procédé selon la revendication 5 ou 6, **caractérisé en ce que** le chauffage est effectué en l'absence de l'oxygène de l'air, pour protéger ainsi les matières végétales d'éventuelles dégradations oxydatives.

8. Procédé selon l'une quelconque des revendications 5 à 7, **caractérisé en ce que** l'étape d'extraction comprend une étape de mise sous vide de la plante, provoquant la vaporisation d'une partie de la plante, cette étape étant réalisée après l'étape de chauffage, à une pression comprise entre 10³ et 2x10⁴ Pa absolus.

9. Procédé selon la revendication 8, **caractérisé en ce que** les vapeurs émises par les plantes lors de la mise sous vide sont condensées.

10. Procédé selon l'une quelconque des revendications précédentes, **caractérisé en ce que** le chauffage est réalisé au moyen d'une vapeur condensante.

11. Procédé selon l'une l'une quelconque des revendications 1 à 9, **caractérisé en ce que** le chauffage est effectué en faisant circuler du jus provenant des plantes traitées, sur les plantes, ou de la vapeur condensée émise lors de la mise sous vide.

12. Procédé selon l'une quelconque des revendications 1 à 9, **caractérisé en ce que** le chauffage est effectué par apport direct de calories aux plantes à partir d'un échangeur approprié, en particulier d'un échangeur à surface raclée.

13. Procédé selon l'une quelconque des revendications précédentes, **caractérisé en ce que** l'étape d'extraction est mise en oeuvre de façon continue ou discontinue.

14. Procédé selon l'une quelconque des revendications précédentes, **caractérisé en ce que** l'étape (b) d'élution met en oeuvre un éluant choisi parmi les solvants alimentaires, et de préférence le méthanol ou l'éthanol.

15. Procédé selon l'une quelconque des revendications précédentes, **caractérisé en en ce que** l'extrait purifié est utilisé sous forme liquide

16. Procédé selon l'une quelconque des revendications 1 à 14, **caractérisé en en ce que** l'extrait purifié est utilisé sous forme sèche

17. Procédé selon la revendication 16, **caractérisé en en ce que** l'extrait purifié est séché par atomisation.

18. Procédé selon la revendication 16, **caractérisé en ce que** l'extrait purifié est séché par lyophilisation.

19. Procédé selon l'une des revendications 16 à 18, **caractérisé en ce que** l'extrait se présente sous la forme de poudre, de granulés ou de billes.

20. Procédé selon l'une quelconque des revendications précédentes, **caractérisé en ce que** l'extrait purifié a une teneur en composés polyphénoliques égale ou supérieure à 30%.

21. Procédé selon l'une quelconque des revendications précédentes, **caractérisé en ce que** les plantes potagères feuillues de la famille des Composées sont choisies parmi la laitue, la chicorée, la frisée et la scarole et les écarts de triage de ces plantes.

22. Procédé selon la revendication 21, **caractérisé en ce que** les plantes potagères feuillues sont des laitues, de préférence choisies parmi la romaine, la batavia et l'iceberg.

23. Procécé selon l'une quelconque des revendications 1 à 20, **caractérisé en ce que** les plantes potagères à bulbe de la famille des Liliacées sont choisies parmi l'oignon, l'ail, le poireau et l'échalote et les écarts de triage de ces plantes.

24. Extrait de salades obtenu par le procédé, selon l'une des revendications 1 à 23 et ayant une teneur en composés polyphénoliques égale ou supérieure à 30%, **caractérisé en ce que** les flavonols représentent au plus 25% des composés polyphénoliques et les esters d'acides hydroxycinnamiques au moins 50% des composées polyphénoliques, par rapport au poids total de l'extrait purifié sec.

25. Extrait selon la revendication 24, **caractérisé en ce que** les esters d'acides hydroxycinnamiques sont des dérivés de l'acide caféique comprenant les esters de l'acide tartrique avec l'acide monocaféoyltartrique, les isomères de l'acide dicaféoyltartrique, l'acide 5'-caféoylquinique (ou acide chlorogénique), l'acide 3'-5'-dicaféoylquinique (ou acide isochlorogénique), l'acide caféoylmalique et l'acide féruoylquinique.

26. Extrait selon la revendication 24, **caractérisé en ce que** les flavonols comprennent le kaempférol-3-glucoside (kaempférol ou lutéoline), le kaempférol-3-glucuronide, le kaempférol-3-O-malonylglucoside, la quercétine-3-glucuronide, la quercétine-3-O-malonylglucoside, les dérivés de kaempférol- malonylglucoside.

27. Extrait selon l'une quelconque des revendications 24 à 26, **caractérisé en ce qu'**il a une activité antiradicalaire définie par un rapport 1/CI₅₀ égal ou supérieur à 0,2 pour une teneur en composés phénoliques égale ou supérieure à 30%, où CI₅₀ est la quantité de produit purifié nécessaire à la réduction de 50% de la quantité de DDPH* présent en une concentration initiale de 6x10⁻⁵mol.l⁻¹.

28. Extrait d'oignons obtenu par le procédé, selon l'une des revendications 1 à 23 et ayant une teneur en composés polyphénoliques égale ou supérieure à 30%, **caractérisé en ce que** les flavonols représentent au moins 80% des composés polyphénoliques par rapport au poids total de l'extrait purifié sec.

29. Extrait selon la revendication 28, **caractérisé en ce que** les flavonols comprennent la quercétine-3,4'-diglucoside, la quercétine-4'-monoglucoside, la quercétine, l'isorhamnétine-3,4'-diglucoside, et l'isorhamnétine-4'-monoglucoside.

30. Extrait selon la revendication 28 ou 29, **caractérisé en ce qu'**il a une activité antiradicalaire définie par un rapport 1/CI₅₀ égal ou supérieur à 0,12 pour une teneur en flavonols égale ou supérieure à 30%, où CI₅₀ est la quantité de produit purifié nécessaire à la réduction de 50% de la quantité de DDPN* présent en une concentration initiale de 6×10⁻⁵mol.l⁻¹.

31. Utilisation d'un extrait purifié à partir de salades ou d'oignons ou d'écarts de triage de ceux-ci tel que défini selon l'une quelconque des revendications 24 à 30 pour la fabrication de produits cosmétiques, d'ingrédients alimentaires, ou de composés pharmaceutiques.

## Claims

1. Method for extracting, fractionating and purifying polyphenolic compounds originating from leafy edible plants of the Compositae family or bulb plants of the Liliaceae family, or from sorting deviations of fresh plants, comprising the following steps of :
a) extracting the polyphenolic compounds in order to obtain a plant raw material extract,
b) adsorbing on an adsorbing resin the polyphenolic compounds contained in the raw extract,
c) eluting the polyphenolic compounds retained on the resin in order to obtain a purified extract, and
d) concentrating, optionally followed by drying, the purified extract in order to obtain a product comprising polyphenolic compounds,
wherein the resin is a styrene-divinyl benzene resin having the following physical features :
1) pores with an average size in the range from 50 to 110 angstroms, preferably from 60 to 100 angstroms,
2) a surface area equal to or higher than 800 m²/g, preferably equal to or higher than 880 m²/g,
3) a pore volume higher than 1 ml/g, and preferably equal to or higher than 1.4 ml/g.

2. Method according to claim 1, wherein the resin has the following physical features :
1) pores with an average size in the range from 80 to 110 angstroms, preferably from 88 to 92 angstroms, and more preferably about 90 angstroms,
2) a surface area equal to or higher than 1000 m²/g, preferably equal to or higher than 1200 m²/g,
3) a pore volume equal to or higher than 2 ml/g, and preferably equal to or higher than 2.4 ml/g.

3. Method according to claim 1 or 2, wherein the adsorption rate of the polyphenolic compounds on the resin is equal to or higher than 80% and the elution rate is equal to or higher than 60%.

4. Method according to anyone of the previous claims, wherein the resin has the formula (I) :

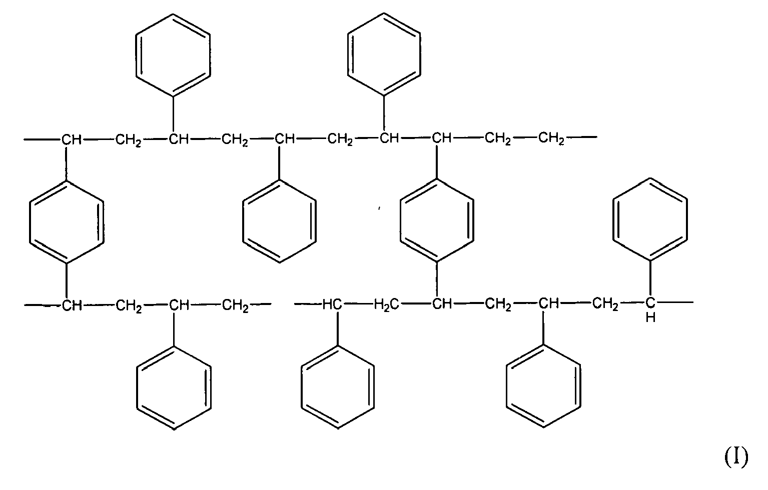

5. Method according to any one of the previous claims, wherein the extraction step (a) prises a quickly heating step of the plant from a room temperature of 25°C up to a temperature as high as 105°C, in order to obtain a hot exudation juice and a must of cooled exuded cooked plants, the exudation juice constituting at least partially the raw extract.

6. Method according to claim 5, wherein the heating is carried out in less than fifteen minutes without maceration.

7. Method according to claim 5 or 6, wherein the heating operation is carried out in the absence of oxygen from the air in order to protect thereby the plant materials against possible oxidative damage.

8. Method according to any one of claims 5 to 7, wherein the extracting step comprises a vacuum step, leading to the vaporisation of a portion of the plant, this step being carried out after the heating step at an absolute pressure in the range from 10³ to 2 x 10⁴ Pa.

9. Method according to claim 8, wherein the vapours originating from the plants during the vacuum step are condensed.

10. Method according to any one of the previous claims, wherein the heating is carried out by using a condensing vapour.

11. Method according to any one of claims 1 to 9, wherein the heating is carried out by circulating juice originating from the treated plants, on the plants, or by circulating condensed vapour emitted during the vacuum step.

12. Method according to any one of claims 1 to 9, wherein the heating is carried out by a direct supply of calories to the plants from an appropriate heat exchanger, in particular, a scraped surface heat exchanger.

13. Method according to any one of claims 1 to 9, wherein the extraction step is carried out either in a continuous or in a batch mode.

14. Method according to any one of claims 1 to 9, wherein the elution step (b) uses an eluent selected from food solvents, and preferably methanol or ethanol.

15. Method according to any one of claims 1 to 9, wherein the purified extract is used in a liquid form.

16. Method according to any one of claims 1 to 14, wherein the purified extract is used in a dry state.

17. Method according to claim 16, wherein the purified extract is spray-dried.

18. Method according to claim 16, wherein the purified extract is freeze-dried.

19. Method according to any one of claims 16 to 18, wherein the extract is a powder, granules or balls.

20. Method according to any one of the previous claims, wherein the purified extract has a polyphenolic compound content equal to or higher than 30%.

21. Method according to any one of the previous claims, wherein the leafy edible plants of the Compositae family are selected amongst lettuce, endive, curly endive and escarole and sorting deviations of such plants.

22. Method according to claim 21, wherein the leafy edible plants are lettuces, preferably selected amongst cos lettuce, batavia and iceberg lettuce.

23. Method according to any one of claims 1 to 20, wherein the bulb edible plants of the Liliaceae family are selected amongst onion, garlic, leek and shallot and sorting deviations of such plants.

24. Lettuce extract obtained by the method according to any one of claims 1 to 23 and having a polyphenolic compound content equal to or higher than 30%, wherein the flavonols represent at the most 25% of the polyphenolic compounds and the hydroxycinnamic acid esters at least 50% of the polyphenolic compounds, based on the total weight of the dry purified extract.

25. Extract according to claim 24, wherein the hydroxycinnamic acid esters consist of caffeic acid derivatives comprising tartaric acid esters with monocaffeoyltartaric acid, isomers of dicaffeoyltartaric acid, 5'-caffeoylquinic acid (or chlorogenic acid), 3'-5'dicaffeoylquinic acid (or isochlorogenic acid), caffeoylmalic acid and feruloylquinic acid.

26. Extract according to claim 24, wherein the flavonols comprise kaempferol-3-glucoside (kaempferol or luteoline), kaempferol-3-glucuronide, kaempferol-3-O-malonylglucoside, quercetine-3-glucuronide, quercetine-3-O-malonylglucoside and kaempferol-malonylglucoside derivatives.

27. Extract according to any one of claims 24 to 26, wherein it has an antiradical activity defined by a 1/CI₅₀ ratio equal to or higher than 0.2 for a phenolic compound content equal to or higher than 30%, where CI₅₀ is the purified product quantity needed for a 50% reduction of DDPH quantity present in an initial concentration of 6 x 10⁻⁵ mole.l⁻¹.

28. Onion extract, obtained by the method according to any one of claims 1 to 23 and having a polyphenolic compound content equal to or higher than 30%, wherein the flavonols represent at least 80% of the polyphenolic compounds based on the total weight of the dry purified extract.

29. Extract according to claim 28, wherein the flavonols comprise quercetine-3,4'-diglucoside, quercetine-4'-monoglucoside, quercetine, the isorhamnetine-3,4'-diglucoside and isorhamnetine-4'-monoglucoside.

30. Extract according to claim 28 or 29, wherein it has an antiradical activity defined by a 1/CI₅₀ ratio equal to or higher than 0.12 for a flavonol content equal to or higher than 30%, where CI₅₀ is the purified product quantity needed for a 50% reduction of DDPH quantity present in an initial concentration of 6 x 10⁻⁵ mole.l⁻¹.

31. Use of a purified extract from lettuces or from sorting deviations of said lettuces, as defined according to any one of claims 24 to 30 for producing cosmetics, food ingredients or pharmaceutical compounds.

## Patentansprüche

1. Verfahren zur Extraktion, Fraktionierung und Reinigung von Polyphenolverbindungen aus Blattgemüsepflanzen der Familie der Korbblütler oder aus Zwiebelgemüsepflanzen der Familie der Liliengewächse oder aus Nebenprodukten von Frischgemüse, umfassend die folgenden Schritte:
a) Extraktion der Polyphenolverbindungen, wobei man einen Gemüserohextrakt erhält;
b) Adsorption der im Rohextrakt enthaltenen Polyphenolverbindungen auf einem adsorbierenden Harz;
c) Elution der auf dem Harz zurückgehaltenen Polyphenolverbindungen, wobei man einen gereinigten Extrakt erhält;
d) Eindicken und dann gegebenenfalls Trocknen des gereinigten Extrakts, wobei man ein an Polyphenolverbindungen reiches Produkt erhält;
**dadurch gekennzeichnet, dass** das Harz ein Styrol-Divinylbenzol-Harz ist, das die folgenden physikalischen Merkmale aufweist:
1) Poren mit einer mittleren Größe von 50 bis 110 Å, vorzugsweise 60 bis 100 Å;
2) eine spezifische Oberfläche von größer oder gleich 800 m²/g, vorzugsweise größer oder gleich 880 m²/g;
3) ein Porenvolumen von über 1 ml/g und vorzugsweise größer oder gleich 1,4 ml/g.

2. Verfahren gemäß Anspruch 1, **dadurch gekennzeichnet, dass** das Harz die folgenden physikalischen Merkmale aufweist:
1) Poren mit einer mittleren Größe von 80 bis 110 Å, vorzugsweise 88 bis 92 Å und besonders bevorzugt ungefähr 90 Å;
2) eine spezifische Oberfläche von größer oder gleich 1000 m²/g, vorzugsweise größer oder gleich 1200 m²/g;
3) ein Porenvolumen von größer oder gleich 2 ml/g und vorzugsweise größer oder gleich 2,4 ml/g.

3. Verfahren gemäß Anspruch 1 oder 2, **dadurch gekennzeichnet, dass** der Adsorptionsgrad der Polyphenolverbindungen auf dem Harz größer oder gleich 80% ist und der Elutionsgrad größer oder gleich 60% ist.

4. Verfahren gemäß einem der vorstehenden Ansprüche, **dadurch gekennzeichnet, dass** das Harz der Formel (I) entspricht:

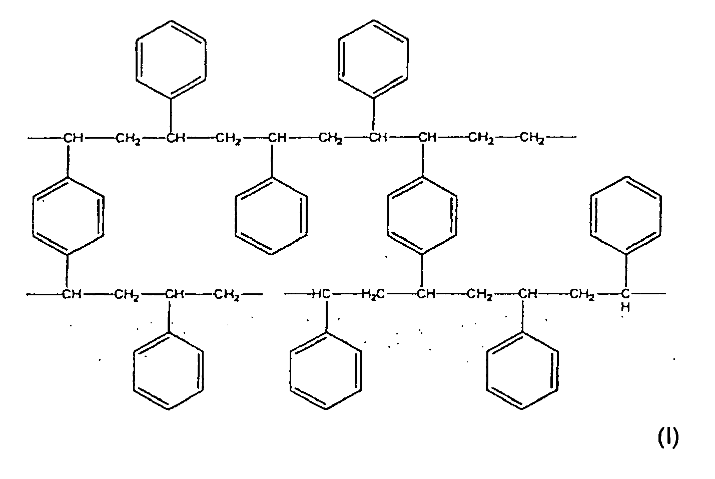

5. Verfahren gemäß einem der vorstehenden Ansprüche, **dadurch gekennzeichnet, dass** der Extraktionsschritt (a) einen Schritt des schnellen Erhitzens der Pflanze von einer Umgebungstemperatur von 25 °C auf eine Temperatur von 105 °C umfasst, wobei man einen heißen Tropfsaft und eine Maische aus abgekühlten ausgelaugten gekochten Pflanzen erhält, wobei der Tropfsaft wenigstens teilweise den Rohextrakt bildet.

6. Verfahren gemäß Anspruch 5, **dadurch gekennzeichnet, dass** das Erhitzen in weniger als fünfzehn Minuten ohne Mazeration erfolgt.

7. Verfahren gemäß Anspruch 5 oder 6, **dadurch gekennzeichnet, dass** das Erhitzen in Abwesenheit von Luftsauerstoff erfolgt, um so die Pflanzenstoffe vor eventuellem oxidativem Abbau zu schützen.

8. Verfahren gemäß einem der Ansprüche 5 bis 7, **dadurch gekennzeichnet, dass** der Extraktionsschritt einen Schritt des Evakuierens der Pflanze umfasst, der die Verdampfung eines Teils der Pflanze hervorruft, wobei dieser Schritt nach dem Schritt des Erhitzens bei einem Druck von 10³ bis 2 x 10⁴ Pa absolut durchgeführt wird.

9. Verfahren gemäß Anspruch 8, **dadurch gekennzeichnet, dass** die beim Evakuieren von den Pflanzen abgegebenen Dämpfe kondensiert werden.

10. Verfahren gemäß einem der vorstehenden Ansprüche, **dadurch gekennzeichnet, dass** das Erhitzen mittels eines kondensierenden Dampfes erfolgt.

11. Verfahren gemäß einem der Ansprüche 1 bis 9, **dadurch gekennzeichnet, dass** das Erhitzen **dadurch** erfolgt, dass man Saft aus den behandelten Pflanzen oder kondensierten Dampf, der beim Evakuieren abgegeben wurde, im Kreislauf auf die Pflanzen zurückführt.

12. Verfahren gemäß einem der Ansprüche 1 bis 9, **dadurch gekennzeichnet, dass** das Erhitzen durch direkten Eintrag von Wärme ausgehend von einem geeigneten Austauscher, insbesondere einem Schabewärmetauscher, auf die Pflanzen erfolgt.

13. Verfahren gemäß einem der vorstehenden Ansprüche, **dadurch gekennzeichnet, dass** der Extraktionsschritt kontinuierlich oder diskontinuierlich durchgeführt wird.

14. Verfahren gemäß einem der vorstehenden Ansprüche, **dadurch gekennzeichnet, dass** im Elutionsschritt (b) ein Eluent eingesetzt wird, der aus lebensmitteltauglichen Lösungsmitteln, vorzugsweise Methanol oder Ethanol, ausgewählt ist.

15. Verfahren gemäß einem der vorstehenden Ansprüche, **dadurch gekennzeichnet, dass** der gereinigte Extrakt in flüssiger Form verwendet wird.

16. Verfahren gemäß einem der Ansprüche 1 bis 14, **dadurch gekennzeichnet, dass** der gereinigte Extrakt in trockener Form verwendet wird.

17. Verfahren gemäß Anspruch 16, **dadurch gekennzeichnet, dass** der gereinigte Extrakt einer Sprühtrocknung unterzogen wird.

18. Verfahren gemäß Anspruch 16, **dadurch gekennzeichnet, dass** der gereinigte Extrakt durch Lyophilisierung getrocknet wird.

19. Verfahren gemäß einem der Ansprüche 16 bis 18, **dadurch gekennzeichnet, dass** der Extrakt in Form eines Puders, Granulats oder von Kügelchen vorliegt.

20. Verfahren gemäß einem der vorstehenden Ansprüche, **dadurch gekennzeichnet, dass** der gereinigte Extrakt einen Gehalt an Polyphenolverbindungen von größer oder gleich 30% hat.

21. Verfahren gemäß einem der vorstehenden Ansprüche, **dadurch gekennzeichnet, dass** die Blattgemüsepflanzen der Familie der Korbblütler aus Gartensalat, Chicorée, Frisée und glatter Endivie und den Nebenprodukten dieser Pflanzen ausgewählt sind.

22. Verfahren gemäß Anspruch 21, **dadurch gekennzeichnet, dass** die Blattgemüsepflanzen Gartensalate sind, die vorzugsweise aus Romana, Batavia und Eisbergsalat ausgewählt sind.

23. Verfahren gemäß einem der Ansprüche 1 bis 20, **dadurch gekennzeichnet, dass** die Zwiebelgemüsepflanzen der Familie der Liliengewächse aus Zwiebel, Knoblauch, Porree und Schalotte und den Nebenprodukten dieser Pflanzen ausgewählt sind.

24. Extrakt von Salaten, der nach dem Verfahren gemäß einem der Ansprüche 1 bis 23 erhalten wurde und einen Gehalt an Polyphenolverbindungen von größer oder gleich 30% hat, **dadurch gekennzeichnet, dass** die Flavonole höchstens 25% der Polyphenolverbindungen ausmachen und die Ester von Hydroxyzimtsäuren wenigstens 50% der Polyphenolverbindungen ausmachen, bezogen auf das Gesamtgewicht des trockenen gereinigten Extraktes.

25. Extrakt gemäß Anspruch 24, **dadurch gekennzeichnet, dass** die Ester von Hydroxyzimtsäuren Derivate der Kaffeesäure sind, die die Ester von Weinsäure mit Monocaffeoylweinsäure, die Isomere von Dicaffeoylweinsäure, 5-Caffeoylchininsäure (oder Chlorogensäure), 3',5'-Dicaffeoylchininsäure (oder Isochlorogensäure), Caffeoyläpfelsäure und Feruloylchininsäure umfassen.

26. Extrakt gemäß Anspruch 24, **dadurch gekennzeichnet, dass** die Flavonole Kaempferol-3-glucosid (Kaempferol oder Luteolin), Kaempferol-3-glucuronid, Kaempferol-3-O-malonylglucosid, Quercetin-3-glucuronid, Quercetin-3-O-malonylglucosid und Derivate von Kaempferolmalonylglucosid umfassen.

27. Extrakt gemäß einem der Ansprüche 24 bis 26, **dadurch gekennzeichnet, dass** er eine Antiradikalwirkung aufweist, die durch ein Verhältnis 1/CI₅₀ von größer oder gleich 0,2 bei einem Gehalt an Phenolverbindungen von größer oder gleich 30% definiert ist, wobei CI₅₀ die Menge des gereinigten Produkts ist, die für die Reduktion von 50% der Menge von in einer Anfangskonzentration von 6 x 10⁻⁵ mol·l⁻¹ vorhandenem DDPH* notwendig ist.

28. Extrakt von Zwiebeln, der nach dem Verfahren gemäß einem der Ansprüche 1 bis 23 erhalten wurde und einen Gehalt an Polyphenolverbindungen von größer oder gleich 30% hat, **dadurch gekennzeichnet, dass** die Flavonole wenigstens 80% der Polyphenolverbindungen ausmachen, bezogen auf das Gesamtgewicht des trockenen gereinigten Extraktes.

29. Extrakt gemäß Anspruch 28, **dadurch gekennzeichnet, dass** die Flavonole Quercetin-3,4'-diglucosid, Quercetin-4'-monoglucosid, Quercetin, I-sorhamnetin-3,4'-diglucosid und Isorhamnetin-4'-monoglucosid enthalten.

30. Extrakt gemäß Anspruch 28 oder 29**, dadurch gekennzeichnet, dass** er eine Antiradikalwirkung aufweist, die durch ein Verhältnis 1/CI₅₀ von größer oder gleich 0,12 bei einem Gehalt an Flavonolen von größer oder gleich 30% definiert ist, wobei CI₅₀ die Menge des gereinigten Produkts ist, die für die Reduktion von 50% der Menge von in einer Anfangskonzentration von 6 x 10⁻⁵ mol·l⁻¹ vorhandenem DDPH* notwendig ist.

31. Verwendung eines aus Salaten oder Zwiebeln oder Nebenprodukten derselben gereinigten Extrakts, wie er gemäß einem der Ansprüche 24 bis 30 definiert ist, zur Herstellung von Kosmetikprodukten, Nahrungsmittelzutaten oder pharmazeutischen Verbindungen.
